# EUROPEAN PATENT APPLICATION

(11) **EP 3 121 600 A1**
(43) Date of publication of application: **25.01.2017**
(21) Application number: 16180462.0
(22) Date of filing: 20.07.2016
(51) Int. Cl.: G01N 33/68

(54) **ANAPHYLAXIS ASSAY**

(30) Priority: 20.07.2015 EP 15177522
(71) Applicant: Worm, Margitta, 10627 Berlin (DE)
(72) Inventor: WORM, Margitta, 10627 Berlin (DE)
(74) Representative: Schulz Junghans Patentanwälte PartGmbB

(57) **Abstract**

The invention relates to a method for diagnosing an anaphylactic reaction / anaphylaxis in a patient, comprising the steps of determining a concentration of 9α,11β-PGF₂ in a sample obtained from said patient; and assigning to said sample a high likelihood of anaphylaxis if said concentration of 9α,11β-PGF₂ is above a predetermined PGF threshold (cut-off), particularly a threshold which is at least two standard deviations above a baseline level determined for a population of control patients, wherein said control patients are not suffering from anaphylaxis or a threshold which is equal to or higher than 170 pg/ml.

## Description

The present invention relates to a method for diagnosing an anaphylactic reaction in a patient.

The problem underlying the present invention is to provide the means for unbiased identification of a patient suffering from an anaphylactic reaction. This problem is solved by the subject-matter of the independent claims.

Anaphylaxis is a severe hypersensitivity reaction with rapid onset. It typically causes a number of symptoms including a rash, itching, throat swelling, low blood pressure, nausea and vomiting. Common triggers of anaphylaxis include insect bites and stings, foods, and medications. On a mechanistic level, anaphylaxis is caused by the release of inflammatory mediators and cytokines from mast cells and basophil granulocytes. Anaphylaxis is potentially life-threatening and requires emergency treatment. Clinicians diagnose the condition on the basis of a patient's symptoms. There is an unmet need for reliable biomarkers to support the clinical diagnosis and to unambiguously identify all anaphylactic responses.

The present invention was made during the course of an investigation assessing the levels of molecules potentially altered during an anaphylactic reaction.

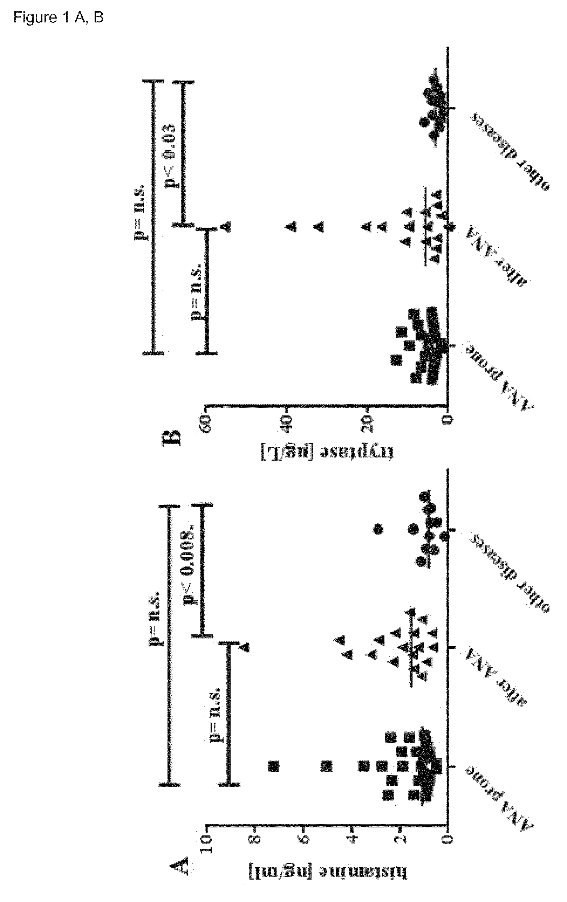

In their study, the investigators examined serum levels of molecules potentially produced by several cell sources, but released in large amounts upon MC activation as biomarkers of ANA. A prior comparison of mediator levels in serum versus plasma revealed no discernable difference between the two blood derived fluids. The cohort consisted of 18 patients sampled ≤2 hrs after an anaphylactic reaction ("after ANA"). Control groups comprised 27 patients sampled outside of an acute reaction, but with a history of ANA ("ANA prone") and 12 patients with acute cardiovascular or febrile reactions ("other diseases").The clinical reaction patterns of the cohorts are specified in Table 1A-C. Focusing on histamine first, the investigators found no difference between the "ANA prone" and the "after ANA" cohort (Fig. 1A), consistent with the short biological half-life of histamine. Anaphylactic episodes may occur anywhere, and a considerable period of time typically passes before the ANA patient is actually seen by an emergency physician. Time seems decisive in the case of histamine, as food allergic patients (n=11) undergoing controlled challenges show slight, yet significant (p= 0.005) increases (maximum increase of 35%) in serum histamine after ≈15 min despite their mild symptoms.

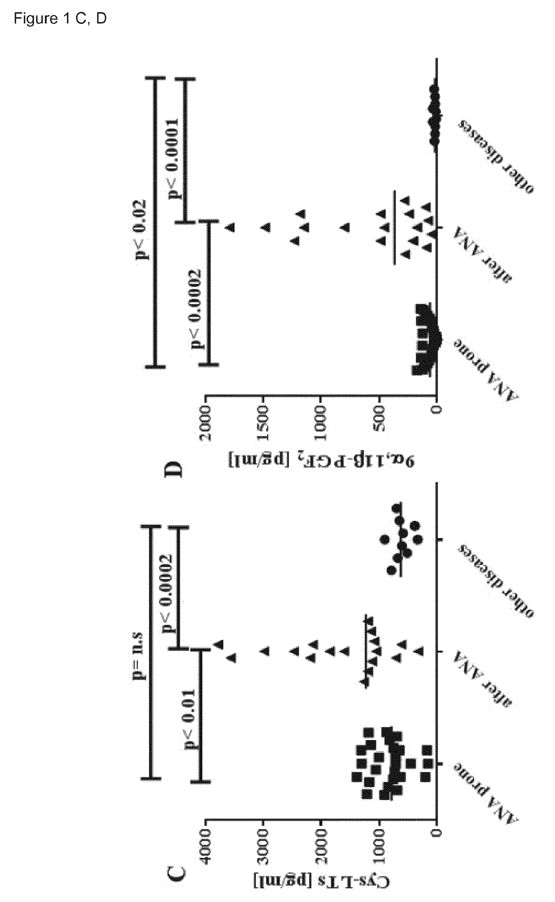

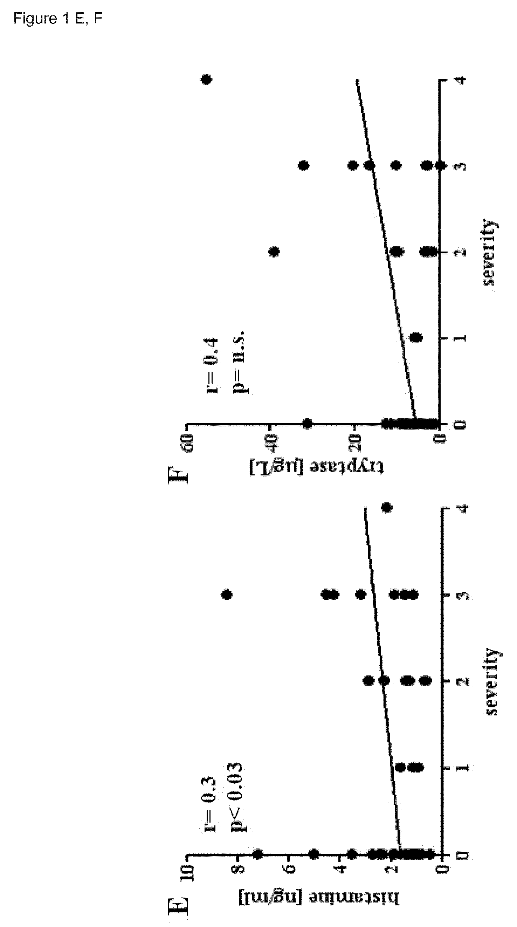

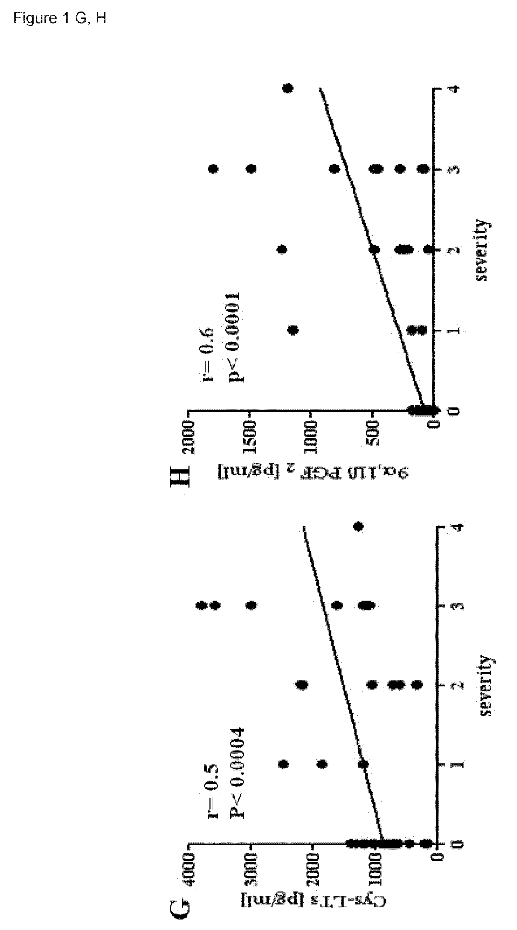

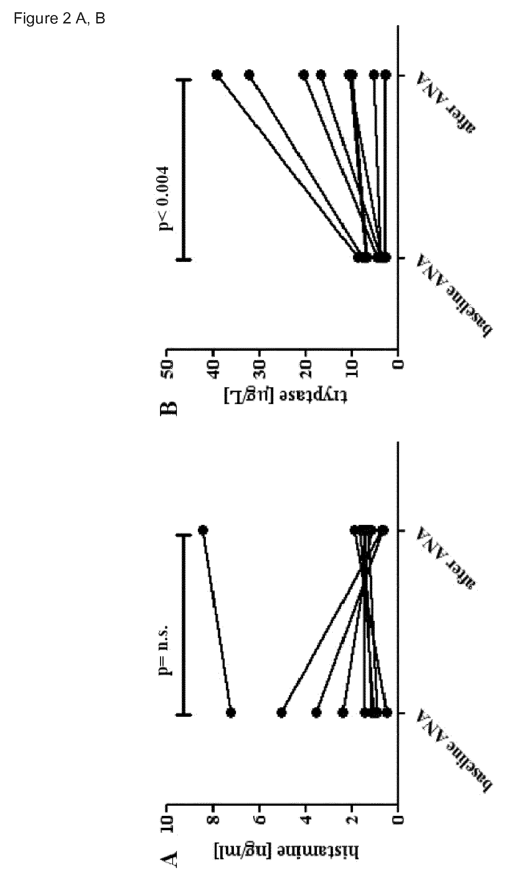

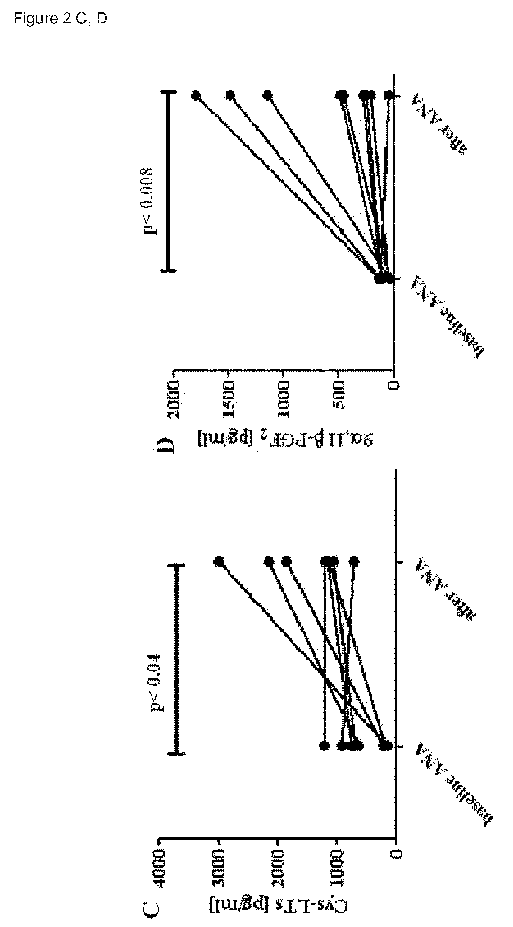

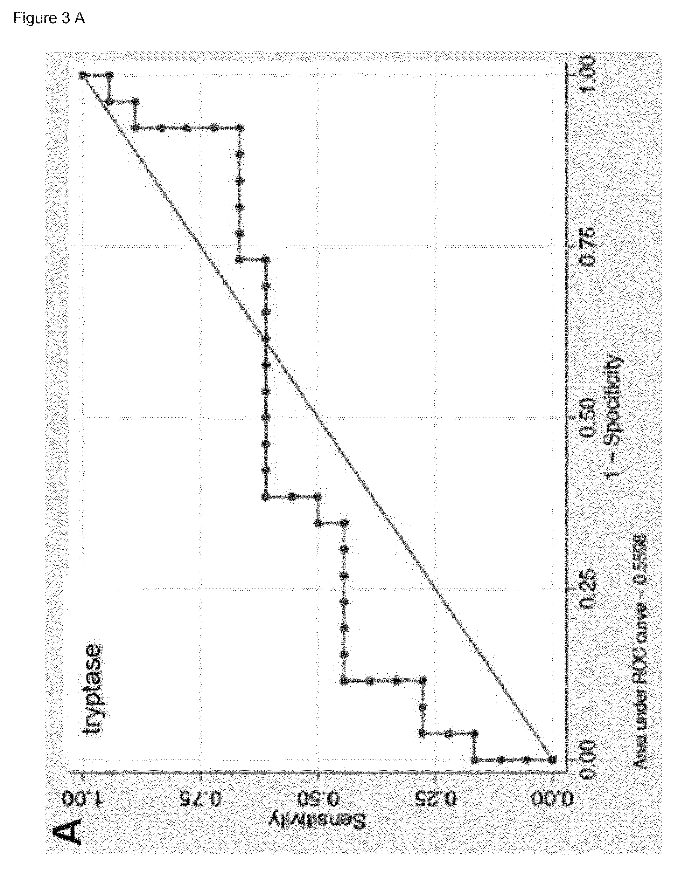

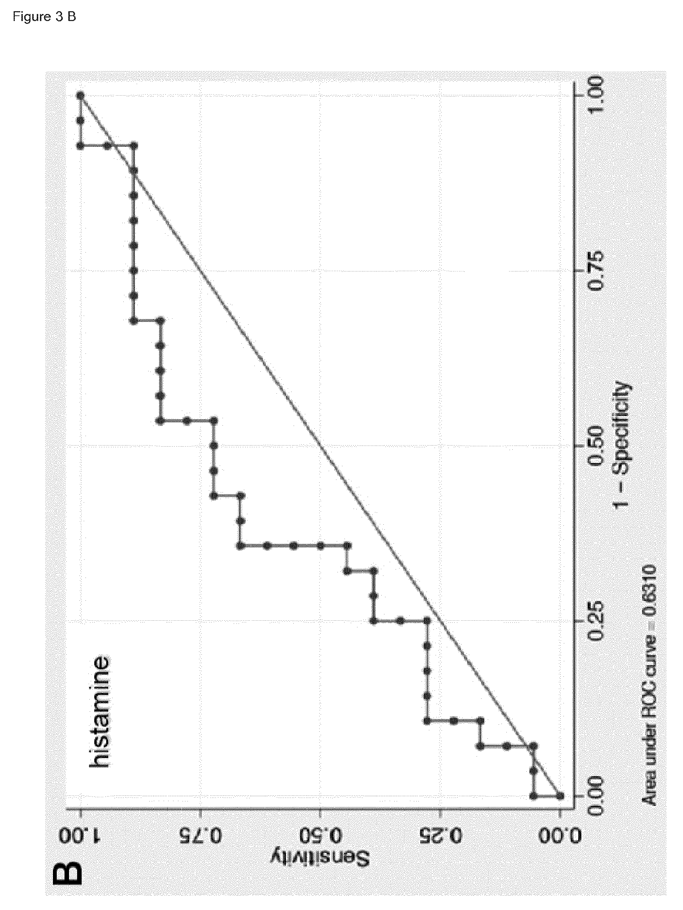

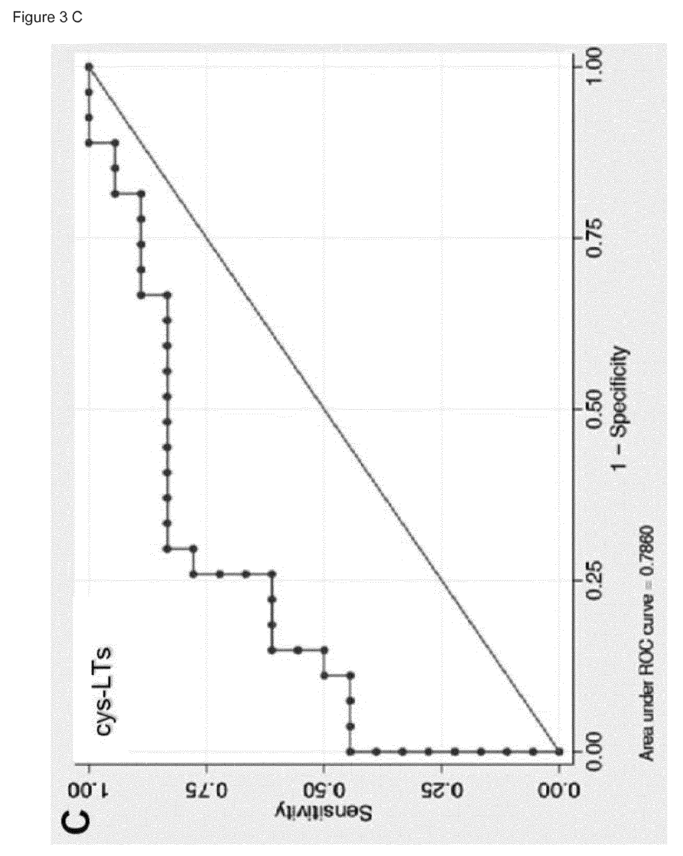

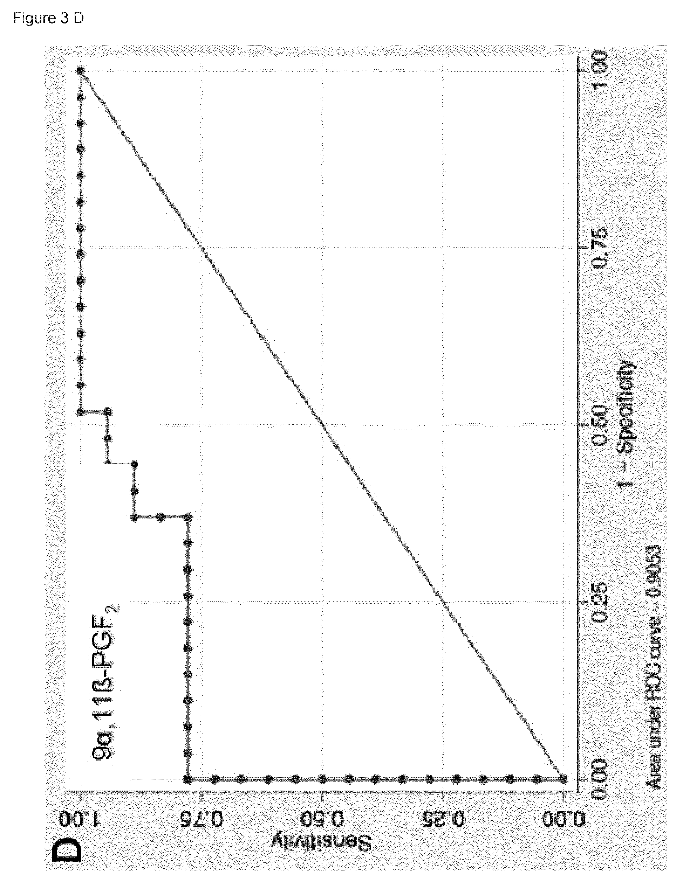

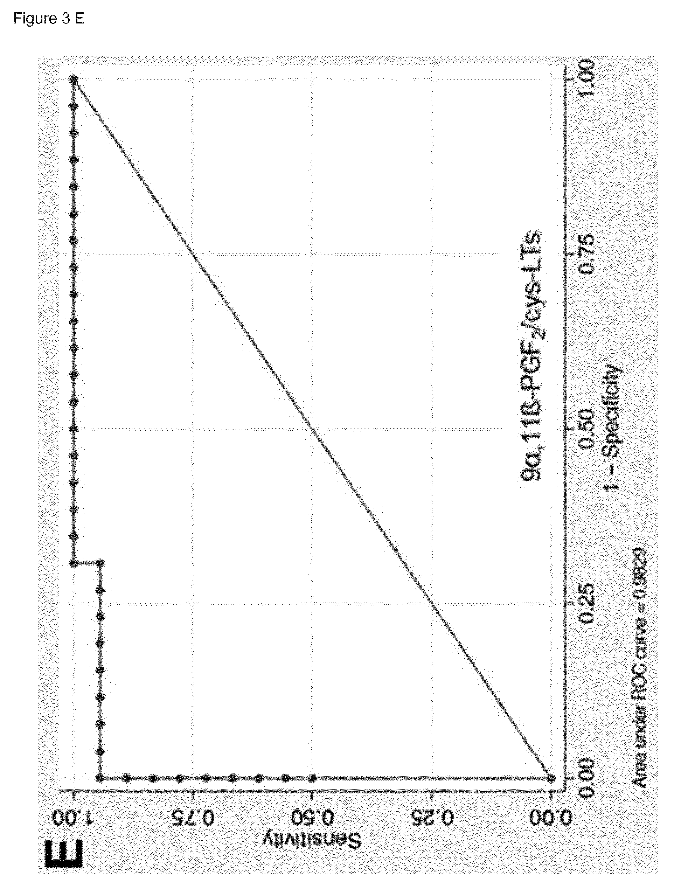

Tryptase is less labile and most sensitively detected at 0.5-2 hrs after ANA. Baseline tryptase levels vary from 1-11 ng/mL in most healthy subjects, but can be elevated in various other conditions, including mastocytosis. In the "after ANA" cohort, the median tryptase level was unaltered compared to that of the ANA-prone group (Fig. 1B), although single values were elevated (>11.4 µg/L). MCs also quickly generate arachidonic acid metabolites upon stimulation, in particular PGD2 and LTC4, that are rapidly metabolized in vivo to 9α,11ß-PGF2 and LTE4, respectively, these metabolites being more stable in body fluids. Their measurements in the context of allergic conditions like anaphylaxis and asthma typically have been reported in urine. When measured in sera, the investigators found significant elevations in cys-LTs (Fig 1C) and 9α,11ß-PGF2 (Fig 1 D), implying their suitability for the detection of ANA in serum up to 2 h after onset of the acute event. Differences between "after ANA" versus "other diseases" were equal or more pronounced if compared with "ANA prone" versus "after ANA", indicating that their elevation was specific for anaphylaxis and not found in other acute conditions (Figure 1). Previous evidence suggested a correlation between histamine or tryptase and the severity of ANA. To examine whether this was true for the cohort under investigation, patients were stratified by severity grade (Ring et al., 1977, Lancet 1:466-9) and mediator levels were plotted in relation to the severity of ANA (Fig. 1E-H). Histamine and tryptase showed little correlation with severity (Fig. 1E,F), and both turned out to be poor biomarkers of ANA by ROC curve analysis using the "after ANA" and the "ANA prone" groups as positive and negative samples, respectively (Fig. 3; Table 2). Conversely, cys-LTs and 9α,11ß-PGF2 showed significant correlation with severity (Fig. 1G,H) and were associated with a higher sensitivity/specificity as measured by AUC values (Fig. 3, Table 2). The preceding analyses concentrated on a comparison between groups of unrelated patients to derive ANA-related mediator profiles even without knowledge of their baseline levels in the patient, since such information may not be available in emergency settings. The investigators wondered whether the above conclusions would prove reproducible if only ANA-triggered changes in the same patient were considered. Matched datasets were available for 9 patients. As illustrated in Fig.2, median concentrations of histamine in the baseline and acute ANA groups were not significantly different, whereas those for acute tryptase, cys-LTs and 9α,11ß-PGF2 were significantly higher than baseline values (Table 3), supporting their use as biomarkers of ANA.

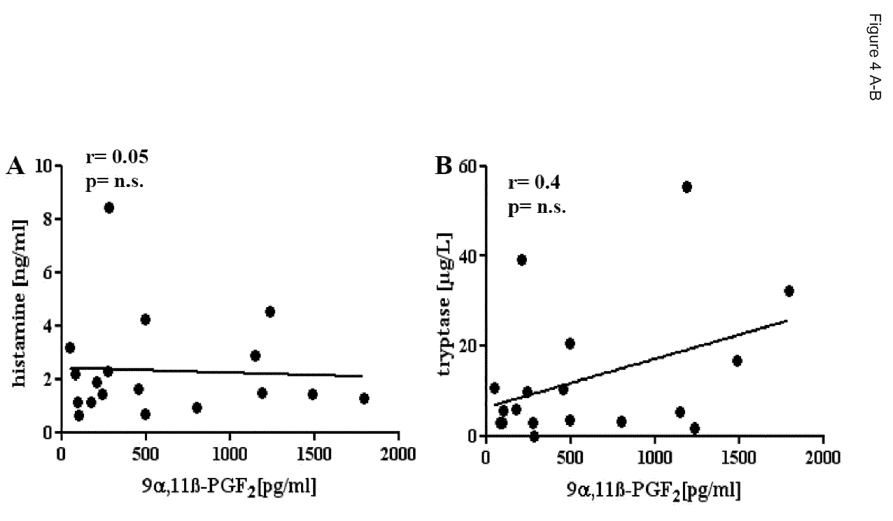

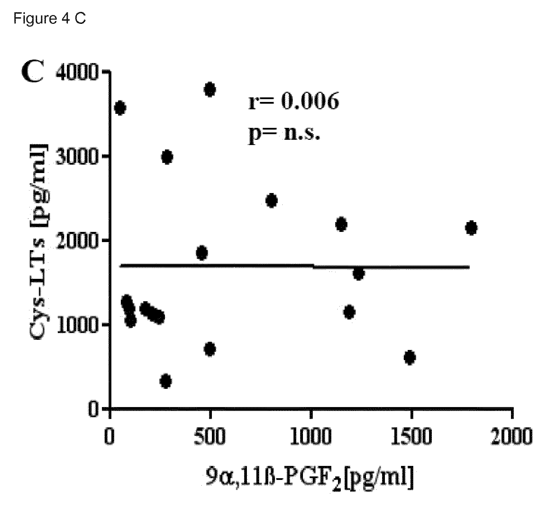

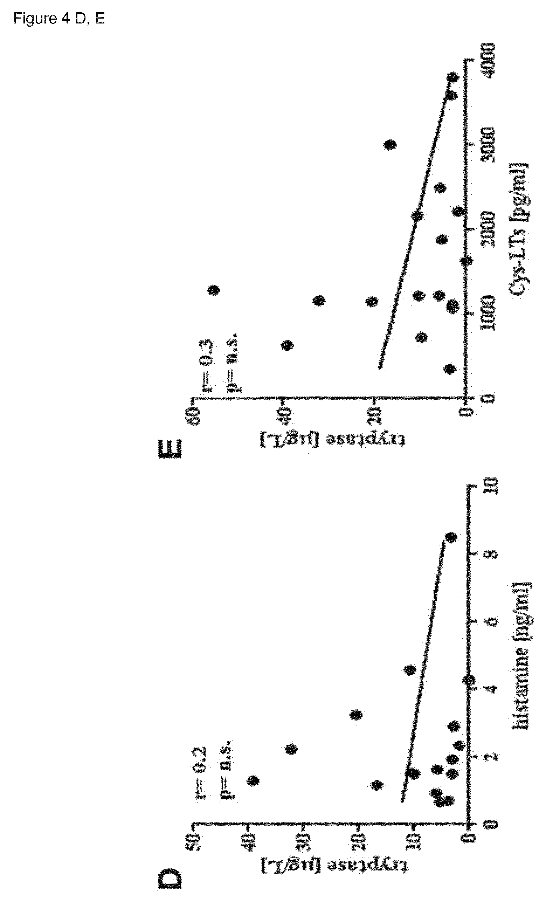

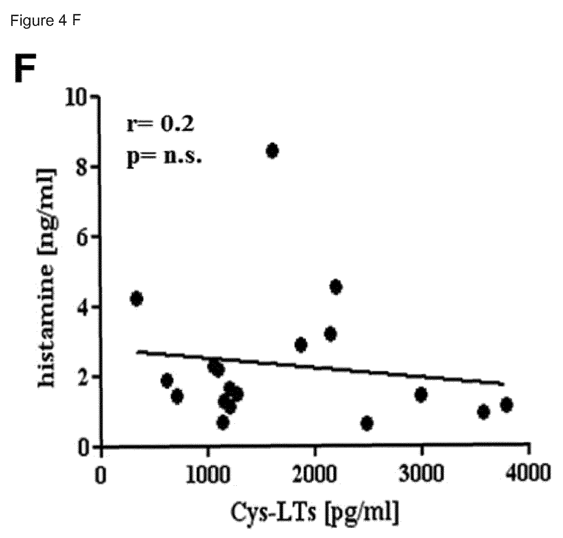

In conclusion the data revealed that the assessed mediators vary in their utility as serum biomarkers of ANA. An appropriate timing of blood withdrawal might increase the sensitivity of each mediator. Histamine seems inadequate under most circumstances due to its short half-life, and may only be able to detect ANA when assessed early after ANA onset. Tryptase is more stable3 and can detect ANA in subgroups of patients, but it is crucial to know the baseline level of the patient, in accordance with previous observations. Metabolites of LTC4 and in particular PGD2 seemed most useful: while the parent mediators are rapidly degraded, cys-LTs and 9α,11ß-PGF2 are more stable and elevated levels are detectable in serum up to 2 hrs after onset of ANA. The particular advantages of 9α,11ß-PGF2 are a) its very low baseline level, b) little variability in the basal concentration among patients, c) its profound increase after ANA (≈8-fold), d) its relative stability and persistence in the circulation, and e) its correlation with severity, making it a robust candidate biomarker. Cys-LTs seem to constitute a supportive marker, but they correlate less well with severity and show more heterogeneous baseline levels. However, because 9α,11ß-PGF2 and cys-LTs are not correlated (pairwise comparisons revealed no significant correlation among the mediators investigated, Fig. 4), the investigators assumed that their combination could result in superior sensitivity/specificity compared to each molecule alone. ROC curve calculations and associated AUC values revealed that a combination of cys-LTs with 9α,11ß-PGF2 indeed improved the diagnostic power of 9α,11ß-PGF2 (Fig 2). The clear increase in serum cys-LTs and 9α,11ß-PGF2 during ANA, combined with their relation to ANA severity and predictive power imply that these molecules will prove as highly suitable serum markers to assist in the diagnosis of ANA.

### Summary of the invention

According to a first aspect of the invention, an *ex vivo*/*in vitro* method for diagnosing an anaphylactic reaction in a patient is provided, comprising the steps of determining the concentration of 9α,11β-PGF₂ in a patient sample and assigning to it a high likelihood of anaphylaxis if the concentration of is above a predetermined threshold.

In certain embodiments, the threshold of 9α,11β-PGF₂ is at least two standard deviations above a baseline level determined for a population of control patients outside an anaphylactic shock reaction or equal or higher (≥) than ≥170 pg/ml or ≥175 pg/ml or 180pg/ml.

In certain embodiments, the obtained sample is a serum sample.

In certain embodiments, the sample is contacted with an antibody specifically binding to 9α,11β-PGF₂.

In certain embodiments, the sample is analyzed by ELISA.

In certain embodiments, the sample was taken 1 h, 2h, 3h, 4h, 5h or 6h after onset of symptoms of anaphylaxis in the patient.

In certain embodiments, the concentration of cys-LTs, particularly cys-Leukotriene C4, cys-Leukotriene D4 and/or cys-Leukotriene E4 is also determined in the sample and a high likelihood of anaphylaxis with increased severity is assigned to the sample if the concentration of 9α,11β-PGF₂ is above the predetermined PGF2 threshold and cys-LT levels are above a predetermined cys-LT threshold.

In certain embodiments, the threshold of cys-LT levels is at least two standard deviations above a baseline level determined for a population of control patients outside an anaphylactic shock reaction or equal or higher (≥) than 1000 pg/ml.

### Terms and definitions

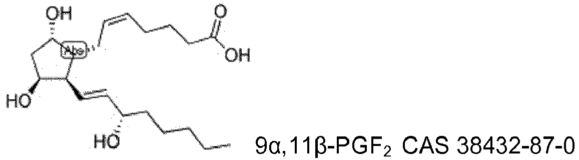

cys-LTs:

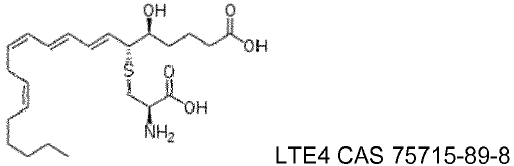

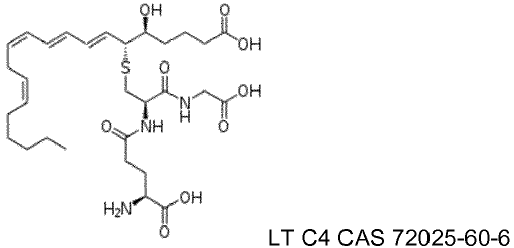

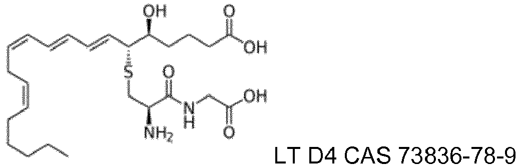

The invention is further illustrated by the following examples and figures, from which further embodiments and advantages can be drawn. These examples are meant to illustrate the invention but not to limit its scope.
Table 1A

**Table E1A Definition of the study cohort "after ANA" and their clinical reaction patterns**

| **number of total cases** | | | **after ANA** | **Mediator** | | | |
|---|---|---|---|---|---|---|---|
| **Elicitor** | **Sex** | **Clinical manifestation** | **Anaphylaxis severity** | **Histamine [ng/ml]** | **Tryptase [µg/L]** | **Cys-LTs [pg/ml]** | **9α,11ß-PGF₂ [pg/ml]** |
| insect | female | skin | I | 1.112 | 5.860 | 1203.026 | 172.504 |
| insect | female | respiratory symptoms cardiovascular symptoms | III | 8.455 | 16.590 | 2991.307 | 1487.829 |
| insect | female | respiratory symptoms skin | III | 1.126 | 10.430 | 1198.554 | 455.371 |
| insect | male | cardiovascular symptoms | IV | 2.203 | 55.370 | 1265.882 | 1183.984 |
| insect | male | skin gastrointestinal symptoms | II | 0.667 | 9.870 | 710.396 | 241.913 |
| insect | male | skin cardiovascular symptoms | II | 1.453 | 39.200 | 618.005 | 208.658 |
| insect | male | skin | I | 1.609 | 5.290 | 186.218 | 1148.530 |
| insect | male | skin cardiovascular symptoms | III | 1,492 | 32.270 | 1143.555 | 1794.455 |
| food | female | skin cardiovascular symptoms | III | 4.530 | 0.00 | 1612.247 | 281.029 |
| food | female | cardiovascular symptoms gastrointestinal symptoms | III | 4.218 | 2.82 | 3789.607 | 91.712 |
| food | female | skin cardiovascular symptoms | III | 1.887 | 20.50 | 1127.916 | 490.787 |
| drugs | female | skin cardiovascular symptoms | II | 0.650 | 2.77 | 1057.900 | 275.765 |
| drugs | female | skin respiratory symptoms | II | 2.880 | 1.75 | 329.087 | 490.848 |
| drugs | female | skin | I | 0.909 | 5.66 | 2479.089 | 99.369 |
| | | | | | | | |

| **number of total cases** | | | **after ANA** | **Mediator** | | | |
|---|---|---|---|---|---|---|---|
| **Elicitor** | **Sex** | **Clinical manifestation** | **Anaphylaxis severity** | **Histamine [ng/ml]** | **Tryptase [µg/L]** | **Cys-LTs [pg/ml]** | **9α,11ß-PGF₂ [pg/ml]** |
| idiopathic | female | skin cardiovascular symptoms | II | 2.880 | 1.75 | 2193.698 | 233.296 |
| idiopathic | male | skin respiratory symptoms | III | 1.452 | 3.00 | 1092.026 | 77.193 |
| idiopathic | female | skin respiratory symptoms | III | 3.204 | 3.16 | 3571.286 | 803.100 |
| idiopathic | female | skin cardiovascular symptoms | II | 1.272 | 10.76 | 2148.403 | 49.673 |

Table 1B

**Table E1B Definition of the study cohort "ANA prone" and their clinical reaction patterns**

| **number of total cases** | | | **ANA prone** | **Mediator** | | | |
|---|---|---|---|---|---|---|---|
| **Elicitor** | **Sex** | **Clinical manifestation** | **Anaphylaxis severity** | **Histamine [ng/ml]** | **Typtase [µg/L]** | **Cys-LTs [pg/ml]** | **9α,11ß-PGF₂ [pg/ml]** |
| insect | female | skin cardiovascular symptoms | II | 1.897 | 5.010 | 1387.000 | 40.394 |
| insect | female | skin cardiovascular symptoms | II | 0.895 | 3.360 | 1306.867 | 79.571 |
| insect | female | skin gastrointestinal symptoms cardiovascular symptoms | III | 0.777 | 3.860 | 821.093 | 24.624 |
| insect | male | skin cardiovascular symptoms respiratory symptoms | III | 1.060 | 3.990 | 728.859 | 67.592 |
| insect | male | skin cardiovascular symptoms | II | 1.014 | 3.730 | 1184.310 | n.d. |
| insect | male | skin cardiovascular symptoms | II | 0.496 | 1.200 | 711.431 | 44.853 |
| insect | male | skin cardiovascular symptoms respiratory symptoms | III | 0.868 | 3.960 | 1172.289 | 81.66 |
| insect | male | skin cardiovascular symptoms | II | 0.780 | 3.250 | 879.616 | 139.434 |
| insect | female | skin respiratory symptoms | II | 0.804 | 3.480 | 760.769 | 5.264 |
| insect | female | skin respiratory symptoms | II | 1.949 | 4.100 | 1146.250 | 133.701 |
| insect | male | cardiovascular symptoms respiratory symptoms | II | 2.325 | 11.600 | 712.862 | 45.300 |
| insect | male | cardiovascular symptoms respiratory symptoms | II | 1.338 | 8.000 | 1061.175 | 26.906 |
| | | | | | | | |

| **number of total cases** | | | **ANA prone** | **Mediator** | | | |
|---|---|---|---|---|---|---|---|
| **Elicitor** | **Sex** | **Clinical manifestation** | **Anaphylaxis severity** | **Histamine [ng/ml]** | **Typtase [µg/L]** | **Cys-LTs [pg/ml]** | **9α,11ß-PGF₂ [pg/ml]** |
| insect | female | skin cardiovascular symptoms | II | 1.626 | 12.900 | 688.621 | 9.902 |
| insect | male | gastrointestinal symptoms cardiovascular symptoms respiratory symptoms | III | 2.749 | 9.600 | 1007.161 | 127.322 |
| insect | male | skin respiratory symptoms | II | 2.487 | 4.500 | 848.779 | 61.276 |
| insect | male | skin cardiovascular symptoms respiratory symptoms | III | 1.248 | 1.900 | 651.540 | 172.058 |
| insect | female | skin respiratory symptoms | II | 0.822 | 5.800 | 1312.411 | 36.916 |
| insect | female | skin respiratory symptoms gastrointestinal symptoms | III | 0.955 | n.d. | 455.874 | 30.957 |
| insect | female | respiratory symptoms cardiovascular symptoms | III | 3.517 | 2.740 | 695.991 | 119.145 |
| insect | male | skin cardiovascular symptoms | III | 0.500 | 4.310 | 175.298 | 102.802 |
| food | female | cardiovascular symptoms gastrointestinal symptoms | III | 7.230 | 3.780 | 155.447 | 123.520 |
| food | female | skin cardiovascular symptoms | III | 2.390 | 6.890 | 1217.910 | 39.542 |
| drugs | female | skin respiratory symptoms | II | 5.040 | 6.770 | 911.958 | 107.594 |
| idiopathic | female | skin cardiovascular symptoms | II | 1.130 | 8.560 | n.d. | 44.454 |
| idiopathic | male | skin respiratory symptoms | III | 1.050 | 3.560 | 203.970 | 41.125 |
| idiopathic | female | skin respiratory symptoms | III | 0.900 | 3.450 | 632.255 | 120.838 |

| **Elicitor** | **Sex** | **Clinical manifestation** | **Anaphylaxis severity** | **Histamine [ng/ml]** | **Typtase [µg/L]** | **Cys-LTs [pg/ml]** | **9α,11ß-PGF₂ [pg/ml]** |
|---|---|---|---|---|---|---|---|
| idiopathic | female | skin cardiovascular symptoms | II | 1.44 | 7.680 | 756.509 | 131.791 |

Table 1C

**Table E1C Definition of the study cohort "other diseases" and their clinical reaction patterns**

| **number of total cases** | | | **Mediator** | | | |
|---|---|---|---|---|---|---|
| **Elicitor** | **Sex** | **Clinical manifestation** | **Histamine [ng/ml]** | **Tryptase [µg/L]** | **Cys-LTs [pg/ml]** | **9α,11ß-PGF₂ [pg/ml]** |
| unknown | female | acute cardiovascular symptoms | 0.722 | 3.91 | 386.557 | 24.796 |
| unknown | male | acute cardiovascular symptoms | 0.162 | 4.01 | 584.497 | n.d. |
| unknown | female | acute cardiovascular symptoms | 1.465 | 2.22 | 609.904 | 21.658 |
| unknown | male | acute cardiovascular symptoms | 2.906 | 2.77 | 682.282 | 13.673 |
| unknown | male | acute cardiovascular symptoms | 0.771 | 601 | 333.551 | 14.711 |
| unknown | male | acute cardiovascular symptoms | 0.871 | 5.1 | 516.491 | 8.79 |
| unknown | female | fever | 1.02 | 1.76 | n.d. | 15.645 |
| unknown | male | fever | 0.463 | 3.57 | 909.616 | 15.725 |
| unknown | female | fever | 1.158 | 3.63 | 791.213 | 36.172 |
| unknown | male | fever | 0.603 | 1.87 | 702.595 | n.d. |
| unknown | female | fever | 0.946 | 1.19 | n.d. | 5.545 |
| unknown | male | fever | 0.81 | 1.88 | 651.415 | 36.353 |

Table 2

**Table E2 Predictive power of mediators to serve as biomarkers for anaphylaxis**

| **Mediator** | **n** | **AUC** | **SID ERR** | **95% CILO** | **95% CI UP** |
|---|---|---|---|---|---|
| Tryptase | 44 | 0.5598 | 0.1020 | 0.3599 | 0.7597 |
| Histamine | 46 | 0.6310 | 0.0859 | 0.4626 | 0.7993 |
| Cys-LTs | 45 | 0.7860 | | 0.6374 | 0.9346 |
| 9α,11ß-PGF₂ | 45 | 0.9053 | 0.0479 | 0.8114 | 0.9993 |
| 9α.11ß-PGF₂/Cys-LTs | 45 | 0.9829 | 0.0178 | 0.9479 | 1.0000 |
| 9α.11ß-PGF₂/Cys-LTs/ tryptase | 42 | 0.9815 | 0.0193 | 0.9436 | 1.0000 |
| 9α,11ß-PGF₂/Cys-LTs/ Histamine | 44 | 0.9829 | 0.0178 0.0193 | 0.9479 | 1.0000 |
| 9α.11ß-PGF₂/Cys-LTs/ Histamine/Tryptase | 42 | 0.9815 | | 0.9436 | 1.0000 |

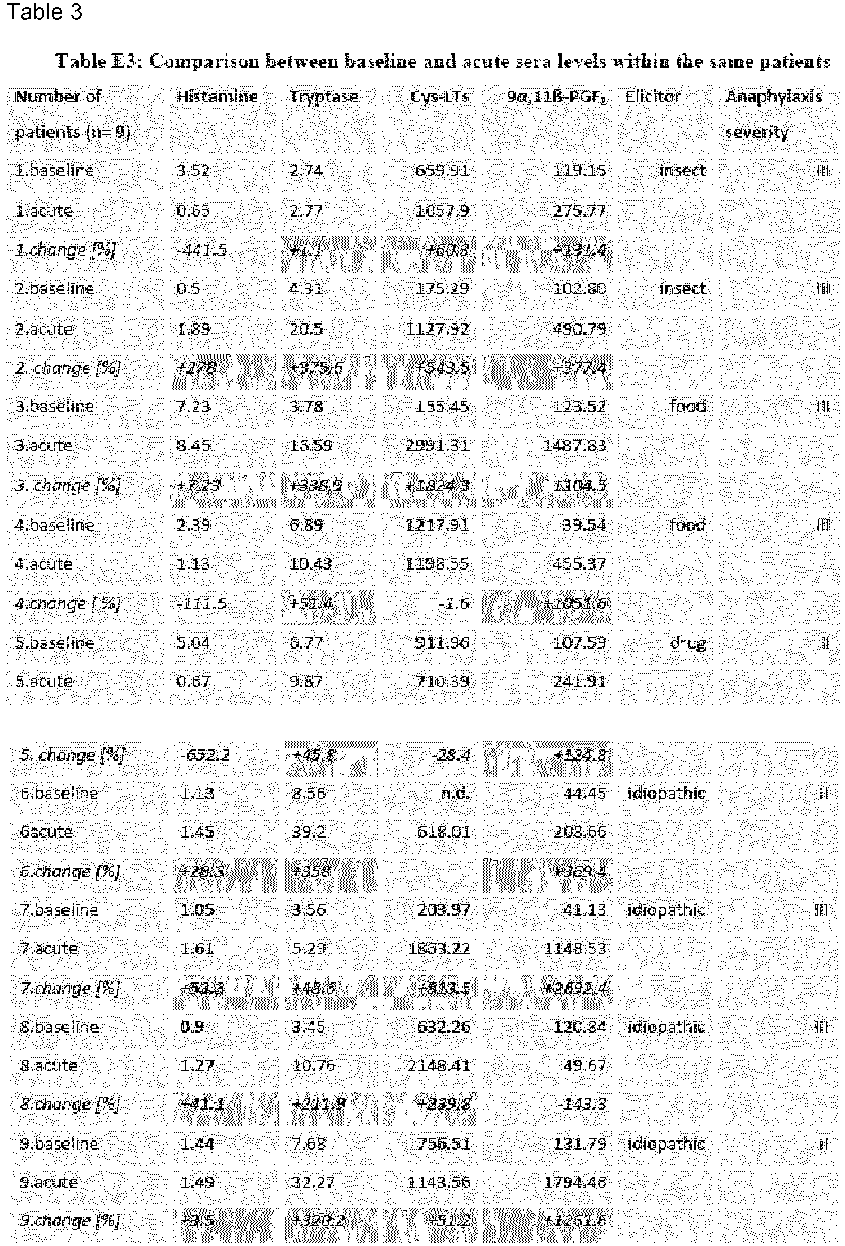

### Description of Figures / Figure Legends

Figure 1: Serum levels of histamine (A), tryptase (B), Cys-LTs (C) and 9α,11ß-PGF2 (D) in sera of acute anaphylactic patients: relation to ANA severity

(A-D) Serum levels of mediators in the active ANA group ("after ANA", blood obtained maximally 2 hrs after the precipitating event) as compared to the control group ("ANA prone") who had experienced ANA in the past, but did not suffer from an anaphylactic reaction at the time of blood withdrawal and a group with acute cardiovascular and febrile reactions ("other diseases"). The cohorts are specified in Table E1A-C in this article's online repository. The mediators were measured by ELISA (histamine, cys-LTs, 9α11ß-PGF2) or by the UniCAP system (tryptase). (E-H) Mediators are shown as a function of anaphylaxis severity. 134 *P < 0.05, **P < 0.01, ***P< 0.001.

Figure 2: Mast cell mediator concentrations during anaphylaxis in a paired setting: the utility of tryptase as a biomarker of ANA depends on comparison with matched non-ANA values 9/18 patients of the "after ANA" group (Fig. 1) provided sera outside of an ANA episode. Mediator concentrations were assessed as in Fig. 1. Dots corresponding to the same patient are inter-connected. *P < 0.05, **P < 0.01, ***P< 0.001.

Figure 3: Predictive power of mediators to serve as biomarkers for anaphylaxis

ROC curves and AUC values are given for: (A) Tryptase, (B) Histamine, (C) cys-LTs, (D) 9α,11ß-PGF2, (E) the combination of 9α,11ß-PGF2 and cys-LTs.

Figure 4: Pairwise correlations between mast cell mediators during anaphylaxis

Correlation coefficients and associated p values for all possible mediator combinations were calculated by Spearman's test. No significant correlations could be detected.

### Table 1A-C

The study compared three groups of patients. (A) The "after ANA" cohort comprised 18 patients admitted to the emergency department in the years 2010-2011 shortly (maximum 2 hours) after an ANA reaction. (B) the control group consisted of 27 patients outside of an acute reaction, but who had suffered at least one ANA episode in the past. The clinical manifestation and severity grades for the latter group refer to that previous ANA episode. The culprit elicitor (if known), sex, manifestation prodfile and ANA grades according to Ring and Messmer and the amount of assessed mediators in sera are given for each patient. (C) The cohort "other diseases" included 12 patients with acute cardiovascular symptoms (n=6) or fever of unknown origin (n=6). Abbreviation: n.d., not detectable

### Table 2

Area under the curves (AUCs) for combinations of mediators were calculated from ROC curves derived by logistic regression analysis. An AUC equal to 1.0 indicates that the diagnostic test discriminates perfectly between anaphylactic and non-anaphylactic response.

### Abbreviation: n.d., not detectable

### Table 3

The amount of histamine, tryptase, cys-LTs and 9α,11ß-PGF2 was detected in basal and acute sera (maximum 2 hrs after an ANA reaction) in the same patient. The amounts detected are followed by a row showing the % change versus baseline. The culprit elicitor (if known) and anaphylaxis severity grades based on Ring and Messmer are given for each patient. % increase in each mediator in acute levels versus baseline levels are indicated in salmon color.

### Methods

We analyzed sera collected from patients with anaphylactic symptoms upon arrival in the emergency departments in 2010-2011 (blood sampled maximally 2 hrs after onset of the reaction) ("after ANA", n= 18). Anaphylaxis was established clinically by routine patient evaluation. Patients were stratified into 4 severity groups according to Ring & Messmer, as based on clinical reaction patterns (Table E1A in this article's online repository). Control groups consisted of patients with a history of anaphylaxis, but without symptoms at the time of blood sampling ("ANA prone", n= 27, Table E1B) and a group with acute cardiovascular or febrile reactions ("other diseases", n=12, Table E1C). Baseline serum samples were available for 9/18 patients from the "after ANA" group and served to compare basal mediator levels with the corresponding acute ANA levels in the same patients. Histamine (LDN, Nordhorn, Germany), cys-leukotrienes (LTC4, LTD4, LTE4) and 9α,11ß-PGF2 (both from Cayman Chemical, Michigan, USA) were measured by ELISA following the manufacturers' instructions. Tryptase levels were quantified by Thermo Fisher Scientific, Freiburg, Germany using the UniCAP-Tryptase fluoroimmunoassay technique. According to the manufacturer, tryptase levels greater than 11.4 µg/L can be regarded as elevated.

### Statistical analysis

Statistical analyses were performed with PRISM 5.0 (GraphPad Software, La Jolla, CA, USA) using non parametrical ANOVA testing with Dunn's post test. Pairwise comparisons were conducted using the Wilcoxon matched pairs test. Correlations were analyzed by Spearman's rank correlation test. Receiver operating characteristic (ROC) curves were 172 constructed, and the area under the curve (AUC) was calculated to establish the predictive power of single mediators as stand-alone diagnostic tools and compared to mediator combinations in an adjunctive setting. An AUC equal to 1.0 indicates that the diagnostic test discriminates perfectly between anaphylactic and non-anaphylactic response, i.e. there are no false positives and no false negatives. An AUC equal to 0.5 indicates that the results of the diagnostic test are purely random. A P value of < 0.05 was considered statistically significant.

## Claims

1. A method for diagnosing an anaphylactic reaction / anaphylaxis in a patient, comprising the steps of
a. determining a concentration of 9α,11β-PGF₂ in a sample obtained from said patient; and
b. assigning to said sample a high likelihood of anaphylaxis if said concentration of 9α,11β-PGF₂ is above a predetermined PGF threshold (cut-off).

2. The method according to claim 1, wherein said predetermined PGF threshold is
a. at least two standard deviations above a baseline level determined for a population of control patients, wherein said control patients are not suffering from anaphylaxis or
b. equal to or higher (≥) than 100 pg/ml, particularly ≥170 pg/ml.

3. The method according to any one of the previous claims, wherein said sample is a serum sample.

4. The method according to any one of the previous claims, wherein said sample is contacted with an antibody specifically binding to 9α,11β-PGF₂.

5. The method according to claim 4, wherein said sample is analyzed by ELISA.

6. The method according to any one of the previous claims, wherein said sample was taken 1h, 2h, 3h, 4h, 5h, or 6h after onset of symptoms in said patient indicative of anaphylaxis.

7. The method according to any one of the previous claims, wherein
a. a concentration of cys-LTs, particularly cys-Leukotriene E4 (CAS No. 75715-89-8), cys-Leukotriene D4 (CAS No. 73836-78-9) and/or cys-Leukotriene C4 (CAS No. 72025-60-6) is determined in said sample and
b. a high likelihood of anaphylaxis with increased severity (severity group 3 or higher according to Ring & Messmer) is assigned to said sample if
i. said concentration of 9α,11β-PGF₂ is above said PGF threshold and
ii. cys-LT levels are above a predetermined cys-LT threshold.

8. The method according to claim 7, wherein said cys-LT threshold is
a. at least two standard deviations above a baseline level determined for a population of control patients, wherein said control patients are not suffering from anaphylaxis or
b. equal to or higher (≥) than 1000 pg/ml.

9. A kit for use in a method according to any one of claims 7 and 8, comprising an antibody specifically binding to 9α,11β-PGF₂ and an antibody specifically binding to cys-Leukotriene E4 (CAS No. 75715-89-8), cys-Leukotriene D4 (CAS No. 73836-78-9) and/or cys-Leukotriene C4 (CAS No. 72025-60-6).
